# EUROPÄISCHE PATENTSCHRIFT

(11) **EP 1 779 327 B1**
(45) Veröffentlichungstag und Bekanntmachung des Hinweises auf die Patenterteilung: **08.06.2011**
(21) Anmeldenummer: 05770222.7
(22) Anmeldetag: 09.08.2005
(51) Int. Cl.: G06T 11/00

(54) **Verfahren und Vorrichtung zur Erzeugung dreidimensionaler tomographischer Bilder eines Objektes**
Method and device for generating three-dimensional images of an object
Procédé et dispositif pour la génération d'images tri-dimensionelles d'un objet

(30) Priorität: 20.08.2004 DE 102004040677; 14.10.2004 DE 102004050172
(43) Veröffentlichungstag der Anmeldung: 02.05.2007
(73) Patentinhaber: Sirona Dental Systems GmbH, 64625 Bensheim (DE)
(72) Erfinder: HEY, Joachim, 53332 Bornheim (DE); LIEVIN, Marc, 53129 Bonn (DE); BREUER, Manfred, 53347 Alfter (DE)
(74) Vertreter: Sommer, Peter
(86) Internationale Anmeldenummer: PCT/EP2005/008613
(87) Internationale Veröffentlichungsnummer: WO 2006/021318

(56) Entgegenhaltungen:
- JIANYING LI: "DIRECT CONE BEAM SPECT RECONSTRUCTION WITH CAMERA TILT" PHYSICS IN MEDICINE AND BIOLOGY, TAYLOR AND FRANCIS LTD. LONDON, GB, Bd. 38, Nr. 2, 1. Februar 1993 (1993-02-01), Seiten 241-258, XP000334472 ISSN: 0031-9155

## Beschreibung

Die vorliegende Erfindung betrifft ein Verfahren zur Erzeugung dreidimensionaler tomographischer Bilder eines Objektes, wobei eine Strahlenquelle, insbesondere eine Röntgenquelle, in Bezug auf das Objekt in einer Bewegungsebene um ein Drehzentrum bewegt wird, wobei die Strahlenquelle ihre Strahlung in einem Strahlenkegel emittiert, dessen Zentralstrahl das Objekt beaufschlagt, wobei im Zentralstrahl auf der der Strahlenquelle abgewandten Seite des Objektes ein entsprechend mitbewegter Detektor angeordnet ist, der von der durch das Objekt in der Intensität geschwächten Strahlung beaufschlagt wird. Die Erfindung betrifft auch eine Vorrichtung zur Umsetzung eines solchen Verfahrens.

Tomographische Aufnahmen sind heutzutage gerade in der Medizin eine wichtige Technik zum Erstellen von Bildern eines durchleuchteten Objekts, respektive des Körpers. Dabei wird das Objekt mit einer Röntgenquelle bestrahlt, die auf einer vorgebbaren Bahn um das Objekt herum bewegt wird. Im Strahlengang hinter dem Objekt ist ein Detektor angeordnet, der ein Array von Detektorelementen aufweist und auf den das Objekt projiziert wird. Die einzelnen Detektorelemente nehmen entsprechende Teilstrahlen der auf den Array auftreffenden und durch Absorption im Objekt geschwächten Röntgenstrahlung auf, wobei aus den Signalen mittels eines Computers ein Bild erstellt wird.

Bei der genannten Technik wird ein Strahl mit konischer Geometrie benutzt, wobei die Röntgenquelle den Lichtkegel auf das Objekt projiziert. Die Detektorelemente werden mit der zweidimensionalen Projektion des Objekts beaufschlagt. Durch die Bewegung von Quelle und Detektor werden Projektionen aus verschiedenen Richtungen gesammelt, wobei die Quelle und der Detektor in fester Anordnung zueinander stehen. Diese Technik ermöglicht die Rekonstruktion des Rauminhalts des durchleuchteten Objekts. In einer besonders bevorzugten Anordnung werden die Röntgenquelle und der Detektor jeweils am Ende eines C-Bogens angeordnet, der um seine Mittelachse um das Objekt herum gedreht wird, wobei eine Vielzahl zweidimensionaler Bilder aufgenommen wird. Aus diesen Projektionen kann das Objekt in seiner Dreidimensionalität rekonstruiert werden. Dabei vollzieht die Strahlenquelle bei den bekannten Kegelstrahltechniken eine kreisförmige oder elliptische Bewegung, wobei der Zentralstrahl senkrecht zur Rotationsachse steht.

Zur Rekonstruktion der dreidimensionalen Objekte wird die Kegelstrahltechnik von Feldkamp genutzt ('Practical cone-beam algorithm' von L.A. Feldkamp, et al. J. Opt. Soc. Am. A/Ausgabe 1, Nr. 6, Juni 1984). Bei dieser Methode, die auch unter dem Begriff "filtered-backprojection" (FBP) bekannt ist, werden alle Projektionsbilder zuerst gefiltert und dann in ihre räumliche Form zurückprojiziert. Die Methode wird in kommerziellen tomographischen Scannern, insbesondere in Spiral-CTs oder Kegelstrahl C-Armen eingesetzt.

Dabei bedürfen derartige Systeme einer Kalibrierung der Geometrie, um die dreidimensionale Darstellung korrekt rekonstruieren zu können. Die Kalibrierung kann online während des Scans oder offline geschehen, wobei die offline Kalibrierung einmalig anhand eines Referenzobjektes (Kalibrierungsdummy) mit bekannter Geometrie ausgeführt wird.

Nachteilig an den bislang bekannten Verfahren ist, dass sie verhältnismäßig empfindlich auf Unregelmäßigkeiten im Objekt, insbesondere bei dentalen Anwendungen, mit Artefakten reagieren. Dabei sei insbesondere die Erstellung von tomographischen Aufnahmen des menschlichen Kiefers genannt, deren Qualität stark von der Verteilung in den Zähnen vorhandener der Metallkronen abhängt. Zudem ist es bei den bekannten Verfahren schwierig, die individuelle Anatomie des Körpers zu berücksichtigen.

Aus dem Aufsatz von Jianying Li: DIRECT CONE BEAM SPECT RECONSTRUCTION WITH CAMERA TILT" Physics in Medicine and Biology, Taylor and Francis Ltd., London, GB, Bd. 38, Nr. 2, 1. Februar 1993, Seiten 241-258, XP000334472 ISSN:0031-9155 ist eine "direct cone beam spect reconstruction" mit schräger Kamera bekannt. Das mit SPECT bezeichnete Verfahren ermöglicht die Erfassung der Verteilung und Anreicherung von Radiopharmaca in einzelnen Organen oder im ganzen Körper mittels Gamma-Detektoren, wobei Schnittbilder erzeugt werden können. Mit Hilfe der radioaktiven Spurenleger werden Stoffwechselwege und -funktionen im Körper verfolgt und bildlich dargestellt. Nachteilig bei diesem Verfahren ist eine nur mäßig gute Ortsauflösung. Aufbauend auf diesen allgemeinen Grundlagen kann der Gamma-Detektor gegenüber einer Rotationsachse schräg gestellt sein. Nachteilig ist, dass ein Positronenemitter injiziert wird, wohingegen bei der vorliegenden Erfindung das Objekt mit Strahlung aus einer Quelle außerhalb des Körpers beaufschlagt wird.

Obwohl eine Kegelstrahlgeometrie mit virtueller Quelle erzwungen wird, ist nicht ersichtlich, Verfahren, die bei SPECT als Verbesserung vorgeschlagen werden, auch ohne weiteres auf CT zu übertragen, da die Größen der Tomogramme um eine Größenordnung auseinander liegen, die statistische Verteilung bei SPECT mäßig bis schlecht und bei CT hingegen sehr gut ist und die tomographische Auflösung bei SPECT im Bereich von 3 mm ist, bei CT hingegen Auflösungen von weit unter 1 mm erreicht werden können.

Aufgabe der Erfindung ist es nunmehr, ein gattungsgemäßes Verfahren zu schaffen, das sich mit einfachen Mitteln kostengünstig umsetzen lässt und das eine hohe Bildqualität bei geringer Strahlenbelastung gewährleistet und dabei die individuelle Anatomie des Körpers berücksichtigen kann. Zudem ist es die Aufgabe der Erfindung, eine mechanisch einfach aufgebaute Vorrichtung zur Umsetzung des Verfahrens zu schaffen.

Diese Aufgaben werden gelöst durch das Verfahren mit den kennzeichnenden Merkmalen des Anspruch 1 und die Vorrichtung nach Anspruch 6. Merkmale besonderer Ausführungsformen der Erfindung sind in den jeweiligen Unteransprüchen genannt.

Der Grundgedanke der Erfindung liegt darin ein Aufnahmeverfahren und den entsprechenden Kegelstrahlapparat zu schaffen, bei dem der Zentralstrahl nicht, wie bislang, in der Bewegungsebene sondern in einem Neigungswinkel gegen die Bewegungsebene geneigt ausgerichtet ist. Da es sich bei der Bewegung in den meisten Fällen um eine Rotation um das zu untersuchende Objekt handelt, ist der Zentralstrahl entsprechend gegen die Rotationsebene geneigt und steht damit nicht mehr senkrecht auf die Rotationsachse.

Dabei wird in einem ersten Fall der Rotationsbewegung, wie bei einem Spiral CT, eine lineare Bewegung entlang der Rotationsachse überlagert. In einem solchen Fall ist die Bewegungsebene geneigt und bildet eine Spirale aus. Erfindungsgemäß schließen der Zentralstrahl und die Rotationsachse auch in diesem Fall einen Winkel ein, der ungleich 90° ist. In weiteren besonderen Fällen, beispielsweise um bei der Aufnahme des Unterkiefers den Schulterbereich auszublenden, kann es vorteilhaft sein, die Ebene der Rotationsbewegung für einen bestimmten Winkelbereich zu verlassen, um dann später wieder in die Ebene zurückzukehren. Die Strahlenquelle und der Detektor machen dabei zunächst eine Aufwärtsbewegung, bevor sie wieder in die alte Bewegungsebene abgesenkt werden. In diesem Fall ist die Bewegung in sich geschlossen. Auch diese speziellen Fälle werden von der Erfindung eingeschlossen.

In einem zweiten Fall wird alternativ oder zusätzlich dazu der Neigungswinkel der Quelle und/oder des Detektors während der Bewegung verändert.

Ein Vorteil besteht im Hinblick auf die Absorption der Strahlungsdosis. So wird es durch die schräge Geometrie möglich, anatomische Strukturen, wie z. B. die Schädelbasis, die besonders sensibel oder stark strahlungsabsorbierend sind, aus dem Strahlengang herauszuhalten. Dadurch werden Messartefakte, wie beispielsweise strahlungsverstärkende Produkte, vermieden, die von diesen anatomischen Strukturen ausgebildet werden. Damit kann die Strahlendosis für den Patienten bei gleicher Bildqualität verringert werden.

Zudem ist es von Vorteil, dass sich mit der Erfindung von Metallobjekten erzeugte Artefakte verringern lassen. So werden solche Metallartefakte gerade bei C-Bögen vermieden, die durch verstärkte Absorption (Okklusionen) entstehen, wie es beispielsweise bei mehreren Zahnfüllung der Fall ist. Dabei können stark absorbierende Objekte die Röntgenstrahlung komplett absorbieren, was sich im aufgenommenen Datensatz wie ein Fehlen von Information auswirkt. Dieser Informationsverlust erzeugt insbesondere dann Artefakte, wenn die klassischen Rekonstruktionsalgorithmen verwendet werden, bei denen der Prozess der Rückprojektion aus einer Summation besteht, wobei im Falle von Okklusionen die Summation inkonsistent ist und die Werte außerhalb des zulässigen Bereichs in eine Sättigung geraten.

Wegen der mathematisch einfacheren Rekonstruktion der Bilder und wegen der leichter interpretierbaren Inhalte der Bilder ist es jedoch besonders vorteilhaft, wenn die Rotationsebene wie bei der herkömmlichen Untersuchung durch einen C-Bogen, senkrecht zur Rotationsachse angeordnet ist und der Bewegungsablauf elliptisch oder kreisförmig ist. Ein weiterer Vorteile dieser Ausführungsform der Erfindung liegt in der einfachen mechanischen Umsetzung. Generell liegen die Vorteile der Erfindung in der geringeren Strahlenbelastung und in der Reduzierung von Artefakten, was zu einer bedeutsamen Steigerung der Bildqualität führt. Im folgenden sind die anatomischen Vorteile für eine dentale Anwendung detailliert dargestellt:

Diese Vorteile sind besonders bemerkenswert bei der Umsetzung der Erfindung im Zusammenhang mit den bekannten C-Bögen, bei denen die Rotationsachse des Systems aus Röntgenquelle und Detektor bislang ausschließlich senkrecht zum zentralen Röntgenstrahl angeordnet war. Die Kalibrierung des Systems ist jedoch ebenso bei der erfindungsgemäß nicht senkrechten Anordnung des Zentralstrahls zur Rotationsachse möglich.

Für die Auswertung ist es vorteilhaft, sich dem Prinzip der genannten Feldkamp-Rekonstruktion zu bedienen, bei dem für die Rekonstruktion der räumlichen Darstellung über alle aus verschiedenen Winkel aufgenommenen Teilstrahlen summiert wird. Dabei ist es wegen des geringen Rechenaufwandes vorteilhaft, Projektionen aus gegenüberliegenden Winkeln (0° und 180°) zu betrachten, die nicht mehr überlagert werden können. Die nicht senkrechte Ausrichtung des Zentralstrahls zur Rotationsachse kann kleine Artefakte produzieren, die auf nicht kompensierte Information während des Rückprojektionsschritts zurückzuführen sind. Experimente haben gezeigt, dass Artefakte durch Metallabsorption viel stärker sind als diejenigen, welche durch nicht kompensierte Informationen erzeugt werden. Daher handelt es sich hier nur um eine geringe Beeinträchtigung.

Es ist vorteilhaft, für C-Bögen die Kegelstrahlrekonstruktion bestimmter Offline-Kalibrierungsdummys zu verwenden ("Kalibrierungsdummys für bildgebende Röntgensysteme" von Mitschke et al., US-Patent 6,715,918). Dieser Dummy ist eine kreisförmige Spirale, welche aus Kugeln zusammengesetzt ist, die einen Binärcode darstellen. Die projizierten Kugeln formen Muster, welche übersetzt werden können. Die Position jeder Kugel ist daher bekannt im dreidimensionalen Raum sowie in zweidimensionalen Bildern. Die Geometrie kann durch Inversion der Projektionsmatrix gefunden werden. Diese Matrizen werden verwendet, um die dreidimensionale Darstellung zu rekonstruieren. Dieser Dummy gestattet es uns, jede Anordnung von Rotationsachse, Mittelstrahl, Detektorfläche unter jeder Kegelstrahlgeometrie zu verwenden.

Nachfolgend wird die Erfindung anhand der Figuren näher erklärt. Es zeigen:
- **Figur 1**: eine schematische Darstellung von Kegelstrahl-Scannersystemen mit Quelle und Detektor,
- **Figur 2**: eine schräge Geometrie eines dentalen Kegelstrahlscanners und
- **Figur 3**: einen Strahl, der durch Zahnfüllungen hindurchgeht.

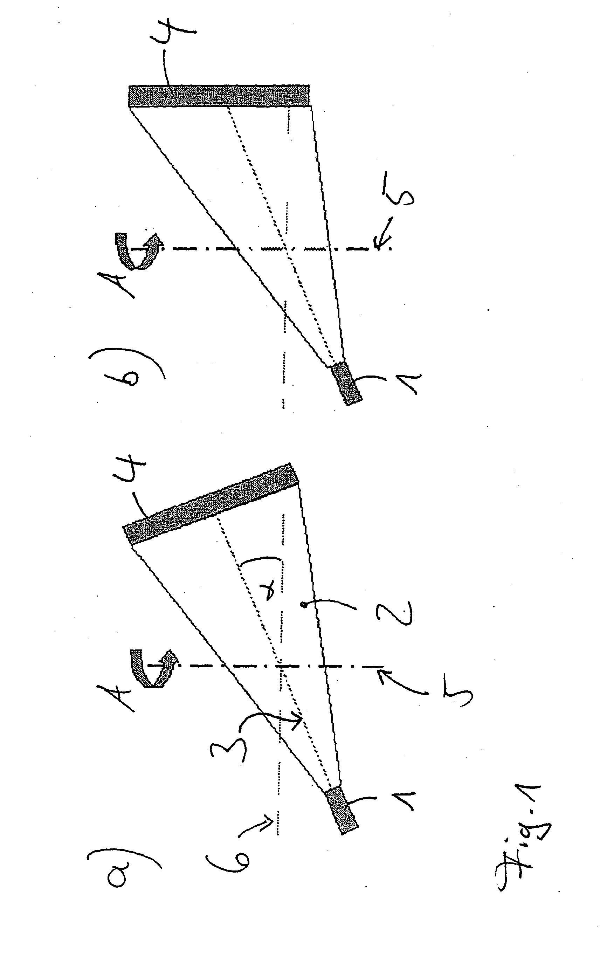

Figur 1 zeigt schematisch ein Kegelstrahl-Scannersystem mit einer Quelle 1 für Röntgenstrahlung. Die Quelle 1 strahlt einen Strahlkegel 2 mit einem Zentralstrahl 3 aus. Nach dem Durchtritt durch ein nicht dargestelltes Objekt trifft der Strahl 2 auf einen Detektorarray 4, der eine Vielzahl einzelner Detektoren aufweist. Jeder der Detektoren nimmt einen durch das Durchleuchten des Objektes geschwächten Teilstrahl des Strahlkegels 2 auf. Zur späteren Erzeugung einer dreidimensionalen Rekonstruktion des Objektes wird die Anordnung aus Quelle 1 und Detektor 4 um eine Achse 5 rotiert (Pfeil A). Die Bewegung der Rotation definiert eine Ebene, die parallel zu der eingezeichneten Ebene 6 ist. Erfindungsgemäß ist der Zentralstrahl 3 gegenüber der Bewegungsebene 6 um einen Winkel α geneigt angeordnet. Der Winkel α kann je nach den Gegebenheiten eingestellt werden. Im Unterschied zu dem Beispiel nach Figur 1a, bei dem der Zentralstrahl 3 den Detektor 4 senkrecht beaufschlagt, ist die Anordnung des Detektors 4 nach Figur 1b parallel zur Drehachse 5. Die beiden Ausführungsbeispiele unterscheiden sich damit lediglich in den Formalismen, die der mathematischen Auswertung zugrunde liegen.

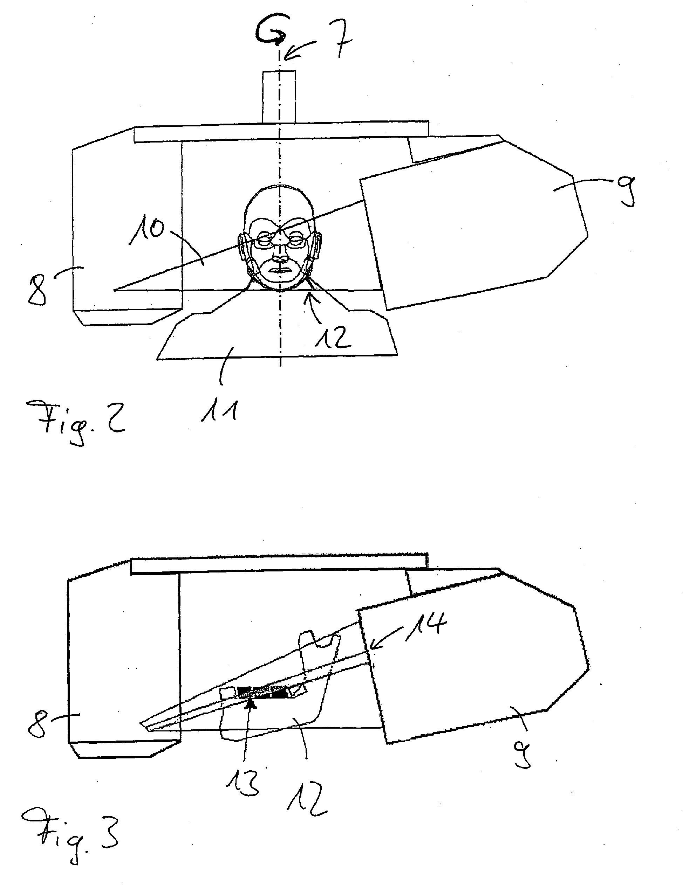

Die Ausführungsform nach Fig. 2 ist nur dann Teil der Erfindung, wenn der Neigungswinkel der Quelle und/oder des Detektors während der Bewegung verändert und/oder wenn das System aus Strahlenquelle und Detektor während der Rotation entlang der Rotationsachse bewegt wird.

Figur 2 zeigt einen um die Achse 7 rotierbaren "C-Bogen" mit Röntgenquelle 8 und schräg angeordnetem Detektor 9. Der Strahlkegel 10 durchleuchtet den unteren Schädel eines Patienten 11. Deutlich zu erkennen ist, dass die Neigung von Quelle 8 und Detektor so gewählt ist, dass der untere Randstrahl 12 horizontal verläuft. Durch diese Anordnung wird die Bestrahlung der Schulter vermieden und der Detektor 9 kann relativ nahe an den Patienten 11 herangeführt werden.

Die Geometrie dieser Einstellung gestattet es einerseits, dass der Detektor die Schulter des Patienten leicht passiert. Dabei kann der Detektor näher an den Patienten herangeführt werden, wodurch das bildgebende Volumen vergrößert werden kann und die Abmessungen des Gerätes optimiert werden können.

In Figur 3 ist die gleiche Anordnung eines "C-Bogens" zu sehen, wobei hier der Unterkiefer 12 des Patienten dargestellt ist. In den Zähnen des Unterkiefers befinden sich metallische Füllungen 13, welche die Strahlung im Bereich 14 abdecken und damit Artefakte in der Rekonstruktion hervorrufen. Deutlich zu erkennen ist jedoch, dass durch die schräge Anordnung nicht alle drei vorhanden Füllungen 13, sondern im Prinzip nur durch eine Füllung gehen. Dadurch wird die verstärkte Absorption vermindert.

## Patentansprüche

1. Verfahren zur Erzeugung 3D-tomographischer Bilder eines Objektes, wobei eine Strahlenquelle (1), insbesondere eine Röntgenquelle, in Bezug auf das Objekt in einer Bewegungsebene (6) um ein Drehzentrum bewegt wird, wobei die Strahlenquelle (1) Strahlung in einem Strahlenkegel (2) emittiert, dessen Zentralstrahl (3) das Objekt beaufschlagt, wobei im Zentralstrahl auf der der Strahlenquelle abgewandten Seite des Objektes ein entsprechend mitbewegter Detektor (4) angeordnet ist, der von der durch das Objekt in der Intensität geschwächten Strahlung beaufschlagt wird,
**dadurch gekennzeichnet,**
**dass** die Bewegung derart durchgeführt wird, dass der Zentralstrahl (3) gegenüber der Bewegungsebene (6) um einen Winkel geneigt ist, und dass der Neigungswinkel der Quelle (1,8) und/oder des Detektors (4,9) während der Bewegung verändert wird und/oder dass das System aus Strahlenquelle (1,8) und Detektor (4,9) während der Rotation entlang der Rotationsachse bewegt wird.

2. Verfahren nach Anspruch 1,
**dadurch gekennzeichnet,**
**dass** der Detektor (4) ein Array von Detektorelementen aufweist, wobei das Objekt während der Bewegung auf den Array projiziert wird und wobei aus den über die Bewegung gesammelten Signale der Detektorelemente ein tomographisches Bild mit dreidimensionaler Information erstellt wird.

3. Verfahren nach Anspruch 1 oder 2,
**dadurch gekennzeichnet,**
**dass** die Strahlenquelle (1) auf der Bahn eines Kegelschnittes, insbesondere auf einer Kreisbahn oder einer Ellipse, um das Objekt herumgeführt wird, wobei der im Strahlengang des Zentralstrahls (3) angeordnete Detektor (4) die Bewegung entsprechend nachvollzieht.

4. Verfahren nach einem der vorherigen Ansprüche,
**dadurch gekennzeichnet,**
**dass** das Detektorarray von zumindest nahezu dem gesamten Strahlenkegel (2) beaufschlagt wird, wobei die aktive Fläche des Detektorarrays senkrecht zum Zentralstrahl (3) angeordnet wird.

5. Verfahren nach einem der vorherigen Ansprüche,
**dadurch gekennzeichnet,**
**dass** das Detektorarray von zumindest nahezu dem gesamten Strahlenkegel (2) beaufschlagt wird, wobei die aktive Fläche des Detektorarrays parallel zur Rotationsachse (5) angeordnet wird.

6. Vorrichtung, insbesondere C-Bogen, zur Durchführung des Verfahrens nach einem der vorherigen Ansprüche, aufweisend eine Röntgenquelle (8) und einen Detektor (9), wobei der Strahlenkegel (10) der Röntgenquelle (8) einen Array von Detektorelementen des Detektors (9) beaufschlagt, wobei die Vorrichtung um eine Rotationsachse (7) drehbar ist,
**dadurch gekennzeichnet,**
**dass** der Zentralstrahl (3) des Strahlenkegels (2,10) gegen die Drehebene geneigt angeordnet ist und dass der Neigungswinkel der Quelle (1,8) und/oder des Detektors (4,9) während der Bewegung veränderbar ist und/oder dass das System aus Strahlenquelle (1,8) und Detektor (4,9) während der Rotation entlang der Rotationsachse bewegbar ist.

7. Vorrichtung nach Anspruch 6,
**dadurch gekennzeichnet,**
**dass** der Neigungswinkel der Quelle (1,8) und/oder des Detektors (4,9) einstellbar ist.

8. Vorrichtung nach Anspruch 6 oder 7,
**dadurch gekennzeichnet,**
**dass** der untere Randstrahl (12) des Strahlenkegels (2,10) bei der Rotation eine horizontale Ebene überstreicht.

## Claims

1. A method for the creation of 3D tomographic images of an object, wherein a source of radiation (1), more particularly a source of X-rays, is moved relatively to said object in a plane of motion (6) about an axis of rotation, during which movement said source of radiation (1) emits radiation as a conical radiation beam (2), whose central ray (3) impinges on said object, while a detector (4) disposed in said central ray is concurrently moved in a corresponding manner on that side of the object that is remote from the source of radiation and the radiation attenuated by said object impinges on said detector (4),
**characterized in that**
said movement is performed in such a manner that said central ray (3) is oblique to said plane of motion (6) by an angle, and that the angle of inclination of said source (1,8) and/or of said detector (4,9) is modified during said movement and/or the system comprising said source of radiation (1,8) and said detector (4,9) is moved along said axis of rotation during rotation.

2. The method as defined in claim 1,
**characterized in that**
said detector (4) has an array of detector elements, and the object is projected onto said array during said movement and a tomographic image with three-dimensional information is created from the signals produced by said detector elements and acquired during said movement.

3. The method as defined in claim 1 or claim 2,
**characterized in that**
said source of radiation (1) is caused to move around said object along the orbit of a conical section, more particularly along a circular or elliptical orbit, and said detector (4) disposed in the optical path of said central ray (3) performs the movement in a corresponding manner.

4. The method as defined in any one of the previous claims,
**characterized in that**
at least almost the entire conical radiation beam (2) impinges on said array of detectors, and the active surface of said array of detectors is at right angles to said central ray (3).

5. The method as defined in any one of the previous claims,
**characterized in that**
at least almost the entire conical radiation beam (2) impinges on said array of detectors, and the active surface of said array of detectors is parallel to said axis of rotation (5).

6. A device, more particularly a C-shaped frame, for carrying out the method as defined in any one of the previous claims, comprising a source of X-rays (8) and a detector (9), wherein the conical radiation beam (10) emitted by said source of X-rays (8) impinges onto an array of detector elements pertaining to said detector (9), said device being rotatable about a axis of rotation (7),
**characterized in that**
said central ray (3) of said conical radiation beam (2,10) is oblique to the plane of rotation and the angle of inclination of said source (1,8) and/or of said detector (4,9) is capable of being modified during said movement and/or the system comprising said source of radiation (1,8) and said detector (4,9) is capable of being moved along said axis of rotation during rotation.

7. The device as defined in claim 6,
**characterized in that**
the angle of inclination of said source (1,8) and/or of said detector (4,9) is capable of being adjusted.

8. The device as defined in claim 6 or claim 7,
**characterized in that**
the lower marginal ray (12) of said conical radiation beam (2,10) sweeps over a horizontal plane during rotation.

## Revendications

1. Procédé de création d'images tomographiques en 3D d'un objet, dans lequel une source de rayons (1), notamment une source de rayons X, est déplacée par rapport à l'objet dans un plan de mouvement (6) autour d'un centre de rotation, la source de rayons (1) émettant un rayonnement dans un cône de rayonnement (2) dont le rayon central (3) touche l'objet, un détecteur (4) se déplaçant en conséquence étant disposé dans le rayon central sur la face détournée de la source de rayons de l'objet et étant touché par le rayonnement dont l'intensité est affaiblie par l'objet,
**caractérisé en ce que**
le mouvement est réalisé de manière à ce que le rayon central (3) soit incliné d'un angle par rapport au plan de mouvement (6) et que l'angle d'inclinaison de la source (1, 8) et/ou du détecteur (4, 9) change pendant le mouvement et/ou que le système composé de la source de rayons (1, 8) et du détecteur (4, 9) se déplace pendant la rotation le long de l'axe de rotation.

2. Procédé selon la revendication 1,
**caractérisé en ce que**
le détecteur (4) présente un réseau d'éléments détecteurs, l'objet étant pendant le mouvement projeté sur le réseau et une image tomographique à information tridimensionnelle étant établie à partir des signaux collectés via le mouvement.

3. Procédé selon la revendication 1 ou 2,
**caractérisé en ce que**
la source de rayons (1) est guidée autour de l'objet sur la piste d'une section conique, notamment sur une piste circulaire ou une ellipse, le détecteur (4) disposé dans le trajet de rayon du rayon central (3) exécutant le mouvement en conséquence.

4. Procédé selon une des revendications précédentes,
**caractérisé en ce que**
le réseau détecteur est touché par au moins pratiquement la totalité du cône de rayonnement (2), la surface active du réseau détecteur étant disposée perpendiculairement au rayon central (3).

5. Procédé selon une des revendications précédentes,
**caractérisé en ce que**
le réseau détecteur est touché par au moins pratiquement la totalité du cône de rayonnement (2), la surface active du réseau détecteur étant disposée parallèlement à l'axe de rotation (5).

6. Dispositif, notamment arc en C, pour la réalisation du procédé selon une des revendications précédentes, présentant une source de rayons X (8) et un détecteur (9), dans lequel le cône de rayonnement (10) de la source de rayons X (8) touche un réseau d'éléments détecteurs du détecteur (9), ce dispositif pouvant tourner autour d'un axe de rotation (7),
**caractérisé en ce que**
le rayon central (3) du cône de rayonnement (2, 10) est disposé incliné vers le plan de rotation et que l'angle d'inclinaison de la source (1, 8) et/ou du détecteur (4, 9) peut être modifié pendant le mouvement et/ou que le système composé de la source de rayons (1, 8) et du détecteur (4, 9) peut se déplacer pendant la rotation le long de l'axe de rotation.

7. Dispositif selon la revendication 8,
**caractérisé en ce que**
l'angle d'inclinaison de la source (1, 8) et/ou du détecteur (4, 9) est réglable.

8. Dispositif selon la revendication 6 ou 7,
**caractérisé en ce que**
le rayon périphérique inférieur (12) du cône de rayonnement (2, 10) passe sur un plan horizontal lors de la rotation.
